# EUROPEAN PATENT APPLICATION

(11) **EP 4 603 528 A1**
(43) Date of publication of application: **20.08.2025**
(21) Application number: 23876514.3
(22) Date of filing: 25.09.2023
(51) Int. Cl.: C08J 3/24, C08L 89/00, C08J 3/075

(54) **COLLAGEN HYDROGEL, PREPARATION METHOD THEREFOR, AND USE THEREOF**

(30) Priority: 14.10.2022 CN 202211262175
(71) Applicant: Suzhou Institute of Nano-tech and Nano-bionics (SINANO) Chinese Academy of Sciences, Suzhou, Jiangsu 215123 (CN)
(72) Inventor: DAI, Jianwu, Suzhou, Jiangsu 215123 (CN); CHEN, Yanyan, Suzhou, Jiangsu 215123 (CN); ZHAO, Haitao, Suzhou, Jiangsu 215123 (CN)
(74) Representative: Ipsilon Lyon
(86) International application number: PCT/CN2023/121026
(87) International publication number: WO 2024/078310

(57) **Abstract**

The present application relates to a collagen hydrogel, a preparation method therefor, and the use thereof. The preparation method comprises the following steps: (1) by using an amine oxidase, catalyzing collagen to undergo oxidative deamination to generate unsaturated aldehyde functional groups, and performing intramolecular or intermolecular crosslinking, so as to complete primary crosslinking; and (2) subjecting the primary cross-linked product to secondary crosslinking under the catalysis of a carboxyl activator so as to obtain the collagen hydrogel. In the present application, the collagen hydrogel prepared by the method is a pure collagen hydrogel, and has a high curing speed, good biocompatibility (excellent bionic properties), adjustable mechanical strength, adjustable structural size, good stability, and a wide range of application.

## Description

### TECHNICAL FIELD

The present application belongs to the technical field of collagen hydrogels, and specifically relates to a collagen hydrogel, a preparation method therefor and use thereof.

### BACKGROUND

Hydrogels have a polymeric network structure and can absorb and retain large amounts of water. In such polymer network, there are hydrophilic groups or regions that can be hydrated under neutral conditions to produce a gel structure. Due to the highly hydrated three-dimensional network, hydrogels provide space for cells in adhesion, proliferation, and differentiation. Therefore, hydrogel scaffolds attract much interest in terms of cell loading and tissue growing.

Collagen, the main component of the extracellular matrix, is the most abundant and widely distributed protein in the animal body. Because of the triple helix structure, collagen not only has excellent mechanical properties, but also has properties such as low antigenicity, blood coagulation effect, easy absorption by the human body and promoting the survival and growth of cells, and has been widely used in the biomedical field.

At present, most collagen hydrogels are prepared by physical cross-linking methods such as low-temperature self-assembly, ultraviolet irradiation, thermal cross-linking, freeze-drying, and hydrogen bonding force, which have the problems of poor mechanical properties and rapid degradation; or collagen has to be combined with other materials to form a composite system to realize gelation, which has the problems of complex composition and unclear degradation products.

CN 109265705A discloses a thiolated collagen derivative, a preparation method therefor and use thereof. By using collagen as a raw material, and using a thiol compound having thiol and carboxyl or having thiol and amino as a modifying agent, a thiol group can be successfully introduced into the collagen molecular chain under the action of a carboxyl activator. The side chain of the disclosed collagen derivative modified by the thiol group is alterable, and thiolated collagen derivatives with different modification can be prepared.

CN 107513172A discloses a preparation method for a collagen membrane; this method uses hydroxycarboxylic acid-N-hydroxysuccinimidyl ester to activate the collagen to form covalent cross-linking, and then uses an oxidase solution for further catalysis to form a secondary cross-linking. The preparation process of this method has the problems such as poor gel flexibility, fast degradation, uncontrollable structure, slow gelation speed and easy distortion, which greatly limits the application of collagen hydrogels, especially fibrous materials, in scaffolds for tissue engineering.

In summary, it is very important to develop a preparation method for a collagen hydrogel with fast curing speed, good mechanical properties, slow degradation rate, controllable structure, and quick and accurate formation.

### SUMMARY

The present application provides a collagen hydrogel, a preparation method therefor and use thereof. The collagen hydrogel prepared by the method is a pure collagen hydrogel with fast curing speed, good biocompatibility (excellent biomimetic performance), adjustable mechanical strength, adjustable structure and size, and good stability, which has a wide application range.

In a first aspect, a preparation method for a collagen hydrogel is provided in the present application, and the preparation method includes the following steps:
(1) catalyzing oxidative deamination of collagen to generate unsaturated aldehyde functional groups by using amine oxidase for intramolecular or intermolecular cross-linking to complete a primary cross-linking; and
(2) subjecting the product from the primary cross-linking to a secondary cross-linking under the catalytic action of a carboxyl activator to obtain the collagen hydrogel.

In the present application, the gelation transformation of pure collagen is achieved through two cross-linking methods: the primary cross-linking (enzymatic cross-linking reaction) and the secondary cross-linking (amidation cross-linking), and the mechanical strength and microstructure of the pure collagen hydrogel are adjusted by changing the concentration of collagen solution, the usage amount of enzyme, and the curing receiving solution.

Specifically, collagen (COL) is the most abundant protein in human and animal bodies. So far, 28 types of collagen have been found in vertebrate bodies, wherein type I collagen is the most abundant and widely used collagen. All collagen molecules are made up of 3 polypeptide chains, i.e., the triple helix structure, which endows collagen with special properties, such as circular dichroism, low antigenicity, and excellent blood coagulation. Collagen is an amphoteric electrolyte, and the basic amino acid in the collagen molecules (such as lysine and histidine) contains ε-amino groups, imino groups and other active groups, which can react with many chemical agents.

In the present application, by using amine oxidase to perform enzyme-catalyzed oxidation of lysyl or hydroxylysyl residue on the collagen peptide chain, lysyl aldehyde or hydroxylysyl aldehyde can be obtained. Further, the chemical cross-linking occurs between aldehyde groups, or between the aldehyde group and active amino group, and the purpose of rapid curing can be achieved.

The method in the present application imitates the maturation mechanism of collagen and elastin in vivo; therefore, the method is also expected to be suitable for the preparation of elastin hydrogels and gelatin hydrogels.

In the present application, the collagen in the collagen solution is pure collagen, which is preferably any one or a combination of at least two of type I collagen from cowhide, type II collagen from cowhide, or type I collagen from rat tail; a typical but non-limiting combination includes a combination of type I collagen from cowhide and type II collagen from cowhide, a combination of type II collagen from cowhide and type I collagen from rat tail, and a combination of type I collagen from cowhide, type II collagen from cowhide and type I collagen from rat tail.

Preferably, the chemical cross-linking reaction in the present application is carried out under weak alkaline conditions, belonging to the Schiff base reaction, pH-responsive chemical bonds, which expands the application scenario.

Preferably, a concentration of the collagen solution is 20-60 mg/mL, for example, 24 mg/mL, 26 mg/mL, 28 mg/mL, 30 mg/mL, 32 mg/mL, 34 mg/mL, 36 mg/mL, 38 mg/mL, 40 mg/mL, 42 mg/mL, 44 mg/mL, 46 mg/mL, 48 mg/mL, 50 mg/mL, 52 mg/mL, 54 mg/mL, 56 mg/mL, or 58 mg/mL.

Preferably, the pH of the collagen solution is less than 7, for example, 6.5, 6, 5.5, or 5.

Preferably, a solvent of the collagen solution includes an organic acid solution.

Preferably, a solvent of the collagen solution includes an acetic acid solution.

Preferably, a mass fraction of the acetic acid solution is 0.01%-30%, for example, 1%, 2%, 5%, 10%, 15%, 20%, or 25%.

Preferably, a concentration of the amine oxidase solution is 5-7 U/mL, for example, 5.2 U/mL, 5.4 U/mL, 5.6 U/mL, 5.8 U/mL, 6 U/mL, 6.2 U/mL, 6.4 U/mL, 6.6 U/mL, or 6.8 U/mL.

In the present application, by adjusting the concentrations of collagen and enzyme, the elastic modulus and the internal pore structure of the pure collagen hydrogel can be adjusted, which expands the application scenario.

Preferably, the pH of the amine oxidase solution is 6.5-7.5, for example, 6.6, 6.8, 7.0, 7.2, or 7.4.

Preferably, the amine oxidase includes any one or a combination of at least two of plasma amine oxidase, monoamine oxidase, diamine oxidase, or lysyl oxidase; a typical but non-limiting combination includes a combination of plasma amine oxidase and monoamine oxidase, a combination of monoamine oxidase, diamine oxidase and lysyl oxidase, and a combination of plasma amine oxidase, monoamine oxidase, diamine oxidase and lysyl oxidase; further preferably, the amine oxidase is plasma amine oxidase.

In the present application, plasma amine oxidase, monoamine oxidase, diamine oxidase, or lysyl oxidase play a similar role in the formation of the collagen fiber in the organism, and plasma amine oxidase is preferred because the studies on plasma amine oxidase are expected to achieve in-situ solidification by using the patient's autologous plasma.

Preferably, the secondary cross-linking is performed in a curing receiving solution containing the carboxyl activator.

Preferably, the pH of the curing receiving solution is 7-9, for example, 7.2, 7.4, 7.6, 7.8, 8, 8.2, 8.4, 8.6, or 8.8.

Preferably, the curing receiving solution includes a weak alkaline solution.

In the present application, the curing receiving solution is a weak alkaline solution containing bicarbonate or carbonate ions. The weak alkaline solution used as the curing receiving solution can provide the reaction conditions for enzymatic cross-linking, and quickly realize the rapid gelation transformation of raw materials to form the first layer of reversible covalent cross-linking network through Schiff base reaction. Simultaneously, the curing solution can react with acetic acid, i.e., the solvent of the collagen solution, to generate carbon dioxide bubbles, and pores are formed by gas-liquid displacement inside the gel.

Preferably, the weak alkaline solution includes any one or a combination of at least two of sodium bicarbonate, potassium bicarbonate, calcium bicarbonate, sodium carbonate or potassium carbonate; a typical but non-limiting combination includes a combination of sodium bicarbonate and potassium bicarbonate, a combination of potassium bicarbonate, sodium carbonate and potassium carbonate, and a combination of sodium bicarbonate, potassium bicarbonate, sodium carbonate and potassium carbonate; further preferably the weak alkaline solution includes sodium bicarbonate (NaHCO₃).

In the present application, the weak alkaline solution is a sodium bicarbonate solution, which avoids collagen denaturation caused by the use of a strong alkaline solution. The weak alkaline solution provides the required weak alkali pH conditions for enzymatic cross-linking, and acetic acid, the solvent of the collagen solution, can quickly react with the sodium bicarbonate solution to generate CO₂ gas inside the hydrogel, then the CO₂ gas converges into bubbles of uniform size, and thereby abundant pores are formed inside the hydrogel after gas-liquid displacement.

Preferably, the primary cross-linking is performed at a temperature of 4-37°C, for example, 5°C, 10°C, 15°C, 20°C, 25°C, 30°C, or 35°C.

In the present application, by mixing the amine oxidase with the collagen solution evenly at room temperature, the amine oxidase can fully exert catalytic oxidation activity.

Preferably, the primary cross-linking is performed for a period of 1 s-12 h, for example, 1 s, 1 h, 2 h, 4 h, 6 h, 8 h, or 10 h.

It should be noted that, in the present application, before the reaction product from the enzyme-catalyzed reaction is added to the curing solution for gelatinization, the preparation method may also include centrifuging the reaction product of the enzyme-catalyzed reaction to remove bubbles caused by external factors, so as to avoid the formation of additional large uneven bubble pores in the gel which affect the internal structure of the gel.

Preferably, the carboxyl activator includes 1-(3-dimethylaminopropyl)-3-ethylcarbodiimide hydrochloride (EDC) and/or N-hydroxysuccinimide (NHS).

In the present application, EDC is a water-soluble carbodiimide, which is used as an activation reagent for carboxyl groups in the synthesis of amide, and is also used for the activation of phosphate groups, cross-linking of proteins and nucleic acids and preparation of immunoconjugates, and is often used in combination with N-hydroxysuccinimide (NHS) or N-hydroxysulfosuccinimide to improve coupling efficiency. The NHS can activate the carboxyl groups, which is conducive to the formation of amide bonds.

The acidic amino acids (such as glutamic acid and aspartic acid) in the collagen peptide chain provide abundant side-chain carboxyl groups, which have strong reactivity, and can react with the remaining amino groups on the collagen peptide chain under the action of the carboxyl activator of EDC/NHS to further form a covalently crosslinked reversible polymer network structure, forming a pure collagen hydrogel.

Preferably, a mass ratio of 1-(3-dimethylaminopropyl)-3-ethylcarbodiimide hydrochloride to N-hydroxysuccinimide is (1-10): 1, wherein the (1-10) may be 2, 3, 4, 5, 6, 7, 8, or 9.

Preferably, a concentration of the carboxyl activator is 0.5-1 mg/mL, for example, 0.6 mg/mL, 0.7 mg/mL, 0.8 mg/mL, or 0.9 mg/mL.

In the present application, the carboxyl activator is further preferably a combination of EDC and NHC having a mass ratio of (1-10):1 and a concentration of 0.5-1 mg/mL; under such condition, an amidation reaction is carried out between the residual amino groups and carboxyl groups in the collagen hydrogel to form a second layer of covalent cross-linking network.

In the present application, optionally, the preparation method further includes gelatinizing the collagen solution by microinjection technology to prepare collagen fibers of different diameters. Specifically, it can be achieved by changing the diameter of the needle for injection squeezing, the flow rate, voltage and other parameters.

In optional embodiments, the collagen hydrogel obtained by covalent cross-linking is washed with water at least 3 times (for example, 4 times, 5 times, or 6 times) to remove residual compounds, and freezing-dried for 2-3 days (for example, 2.2 days, 2.4 days, 2.6 days, or 2.8 days) to obtain the dried pure collagen hydrogel.

In the above preparation process, firstly, amine oxidase is used to catalyze the oxidation of collagen to carry out an enzymatic cross-linking reaction to achieve the rapid curing of the pure collagen hydrogel; secondly, the carboxyl activator EDC/NHS is used for amidation cross-linking to further enhance the mechanical properties of the pure collagen hydrogel.

Preferably, the secondary cross-linking is performed at a temperature of 4-37°C, for example, 5°C, 10°C, 15°C, 20°C, 25°C, 30°C, or 35°C.

Preferably, the secondary cross-linking is performed for a period of 0.1-12 h, for example, 1 h, 2 h, 4 h, 6 h, 8 h, or 10 h.

As a preferred technical solution, the preparation method includes the following steps:
(1) subjecting a collagen solution with a concentration of 20-60 mg/mL and pH of less than 7 and an amine oxidase solution with a concentration of 5-7 U/mL and pH of 6.5-7.5 to oxidative deamination by enzyme catalyzing at 4-37°C for 1 s-12 h to complete a primary cross-linking; and
(2) subjecting the product from enzyme-catalytic reaction to a secondary cross-linking at 4-37°C for 1 s-12 h in a curing receiving solution containing a carboxyl activator with pH of 7-9 to obtain the collagen hydrogel.

In the present application, the method consists of two steps chronologically: the first step is the Schiff base reaction mediated by the amine oxidase, which enables the hydrogel to be rapidly cured within a few seconds, meeting the requirements of precision manufacturing and rapid formation, and expanding the application of the hydrogel in the fields of dry/wet spinning and 3D bioprinting; however, Schiff base is sensitive to pH while improving the fluidity of molecular chains in the hydrogel, and in order to increase the stability of the hydrogel, a second step of amidation coupling mediated by the carboxyl activator is carried out to enhance the stability of the hydrogel.

In a second aspect, the present application provides a collagen hydrogel, and the collagen hydrogel is prepared by the preparation method as described in the first aspect.

In a third aspect, the present application provides use of the collagen hydrogel as described in the second aspect in biomedical materials or scaffolds for tissue engineering.

In the present application, the collagen hydrogel imitates the formation mechanism of collagen fibers in vivo, and has excellent biomimetic performance and biocompatibility.

Compared with the prior art, the present application has the following beneficial effects.
(1) In the present application, the collagen hydrogel prepared by the method is a pure collagen hydrogel with fast curing speed, good biocompatibility (excellent biomimetic performance), adjustable mechanical strength, adjustable structure and size, and good stability, which has a wide application range.
   The collagen hydrogel in the present application has a maximum elongation at break of 79.13 ± 2.54% or more, a maximum tensile stress of 118.60 ± 2.3 kPa or more, and a fiber diameter within the range of 199.59 ± 8.45 - 860.91 ± 15.44 µm. The collagen hydrogel in the present application has good mechanical strength and elastic properties, and its fiber diameter is small.
(2) In the present application, compared with the current pure physical collagen hydrogel, multicomponent blended collagen hydrogel, etc., the collagen hydrogel prepared by the method not only improves the curing speed (the gelatinization in the present application can be realized within 12 s, and the prior art needs more than 6 min), but also improves the mechanical properties and stability of the collagen hydrogel, and the composition of the gel is simple and clear, and the collagen hydrogel can be applied to 3D bioprinting and additive manufacturing.
(3) In the present application, compared with the currently developed pure collagen hydrogel prepared from cross-linking by EDC/NHS carboxyl activator first and then by enzyme, the curing speed is improved, and abundant pores can be formed with the help of a large number of dense CO₂ bubbles generated inside the collagen hydrogel by the reaction between solvents. Meanwhile, the enzymatic cross-linking causes reversible changes in the conformation of the collagen molecular chain, which increases the fluidity of the molecular chain, and finally leads to good flexibility of the collagen hydrogel fiber. In addition, the size of hydrogel fiber is controllable, on the basis of which various material scaffolds can be constructed.

### BRIEF DESCRIPTION OF DRAWINGS

Fig. 1 shows an actual object diagram of the bulk collagen hydrogel prepared by the method in Example 1.
Fig. 2 shows an actual object diagram of the collagen hydrogel fiber prepared by the method in Example 1.
Fig. 3 shows infrared results of the collagen, product from enzymatic cross-linking reaction and collagen hydrogel fiber involved in the method in Example 1.
Fig. 4 shows a microscopic cross-sectional image of the collagen hydrogel fiber prepared by the method in Example 1.
Fig. 5a shows a surface morphology image of the collagen hydrogel fiber prepared by the method in Example 1.
Fig. 5b shows a surface morphology image of the collagen hydrogel fiber prepared by the method in Example 2.
Fig. 5c shows a surface morphology image of the collagen hydrogel fiber prepared by the method in Example 3.
Fig. 5d shows a surface morphology image of the collagen hydrogel fiber prepared by the method in Example 4.
Fig. 6 shows a stress-strain diagram of the collagen hydrogel fibers prepared by the methods in Example 1, Example 5 and Example 6.
Fig. 7 shows a stress-strain diagram of the collagen hydrogel fibers prepared by the methods in Example 1, Example 7 and Example 8.
Fig. 8 shows a chart of time-sweep test of the gelatinization process of the method in Example 13.
Fig. 9 shows a chart of time-sweep test of the gelatinization process of the method in Comparative Example 1.
Fig. 10 shows an image demonstrating the collagen hydrogel fiber prepared by the method in Example 1 used in scaffold for tissue engineering.

### DETAILED DESCRIPTION

Embodiments of the present application are further described below for a better understanding of the present application. It should be clear to those skilled in the art that the embodiments are merely used for a better understanding of the present application and should not be regarded as a specific limitation to the present application.

In the present application, macromolecular collagen used in each embodiment is extracted from cowhide collagen; the main component is type 1 collagen.

### Example 1

This example provides a preparation method for a collagen hydrogel, which includes the following steps:
(1) 40 mg pure collagen was dissolved in 1 mL of an aqueous solution of acetic acid with a mass fraction of 1%, and fully dissolved in a water bath at 37°C and an ultrasonic oscillator until a transparent solution was formed to form a collagen solution;
(2) 20 mg plasma amine oxidase was accurately weighed out and added with 1 mL of double distilled water and fully dissolved, and the solution was subpackaged and stored in dark at -20°C; the formed amine oxidase solution had pH of 7 and a concentration of 6.8 U/mL;
(3) 10 µL of the above plasma amine oxidase solution was taken and added to 1 mL of the fully dissolved collagen solution, the mixture was evenly mixed, subjected to an enzyme-catalyzed primary cross-linking reaction at 25°C for 30 min, then centrifuged to remove bubbles and sucked into a 1 mL syringe (the size of the syringe was 25 G) of for later squeezing;
(4) the product of the above enzymatic cross-linking reaction was squeezed into a pre-cooled NaHCO₃ aqueous solution containing 50wt% of EDC:NHS (4:1), and cured at 25°C for 30 min to complete a secondary cross-linking to obtain a double-crosslinked pure collagen hydrogel fiber; and
(5) the above crosslinked collagen hydrogel was immersed in double distilled water and washed for 3 times to obtain the collagen hydrogel fiber.

### Examples 2-4

Examples 2-4 differ from Example 1 in that the syringes were 27 G (Example 2), 22 G (Example 3) and 18 G (Example 4) in size. Others were the same as those of Example 1.

### Example 5

This example provides a preparation method for a collagen hydrogel, which includes the following steps:
(1) 30 mg pure collagen was dissolved in 1 mL of a solution of acetic acid with a mass fraction of 1%, and fully dissolved in a water bath at 37°C and an ultrasonic oscillator until a transparent solution was formed to form a collagen solution;
(2) 20 mg plasma amine oxidase was accurately weighed out and added with 1 mL of double distilled water and fully dissolved, and the solution was subpackaged and stored in dark at -20°C; the formed amine oxidase solution had pH of 7 and a concentration of 6.8 U/mL;
(3) 10 µL of the above plasma amine oxidase solution was taken and added to 1 mL of the fully dissolved collagen solution, the mixture was evenly mixed, subjected to an enzyme-catalyzed primary cross-linking at 25°C for 30 min, then centrifuged to remove bubbles and sucked into a 1 mL syringe for later squeezing;
(4) the above collagen solution was squeezed into a pre-cooled NaHCO₃ solution containing 50wt% of EDC:NHS (4:1), and cured at 25°C for 30 min to complete a secondary cross-linking to obtain a double-crosslinked pure collagen hydrogel fiber; and
(5) the above crosslinked collagen hydrogel was immersed in double distilled water and washed for 3 times to obtain the collagen hydrogel fiber.

### Example 6

This example provides a preparation method for a collagen hydrogel, which includes the following steps:
(1) 50 mg pure collagen was dissolved in 1 mL of a solution of acetic acid with a mass fraction of 1%, and fully dissolved in a water bath at 37°C and an ultrasonic oscillator until a transparent solution was formed to form a collagen solution;
(2) 20 mg plasma amine oxidase was accurately weighed out and added with 1 mL of double distilled water and fully dissolved, and the solution was subpackaged and stored in dark at -20°C; the formed amine oxidase solution had pH of 7 and a concentration of 6.8 U/mL;
(3) 10 µL of the above plasma amine oxidase solution was taken and added to 1 mL of the fully dissolved collagen solution, the mixture was evenly mixed, subjected to an enzyme-catalyzed primary cross-linking reaction at 25°C for 30 min, then centrifuged to remove bubbles and sucked into a 1 mL syringe for later use;
(4) the above collagen solution was squeezed into a pre-cooled NaHCO₃ solution containing 50wt% of EDC:NHS (4:1), and cured at 25°C for 30 min to complete gelatinization and a secondary cross-linking to obtain a double-crosslinked pure collagen hydrogel fiber; and
(5) the above crosslinked collagen hydrogel was immersed in double distilled water and washed for 3 times to obtain the collagen hydrogel fiber.

### Examples 7 and 8

Examples 7 and 8 differ from Example 1 in that the mass of the pure collagen was 20 mg (Example 7) and 60 mg (Example 8), and the mass concentration of the formed collagen solution was 20 mg/mL and 60 mg/mL, respectively. Others were the same as those of Example 1.

### Examples 9 and 10

Examples 9 and 10 differ from Example 1 in that the concentration of the plasma amine oxidase was 4 U/mL (Example 9) and 8 U/mL (Example 10). Others were the same as those of Example 1.

### Example 11

This example differs from Example 1 in that step (4) specifically was: the product of the above enzymatic cross-linking reaction was squeezed into a pre-cooled NaHCO₃ solution containing 50wt% of EDC:NHS (4:1), and cured at 4°C for 30 min to complete gelatinization and a secondary cross-linking to obtain a crosslinked pure collagen hydrogel fiber. Others were the same as those of Example 1.

### Example 12

This example differs from Example 1 in that NaHCO₃ was not included in step (4). Others were the same as those of Example 1.

### Example 13

This example differs from Example 1 in that NaHCO₃ and EDC/NHS were not included in step (4). Others were the same as those of Example 1.

### Comparative Example 1

This comparative example differs from Example 1 in that the secondary cross-linking was not performed. Others were the same as those of Example 1.

### Comparative Example 2

This comparative example differs from Example 1 in that only the secondary cross-linking was performed. Others were the same as those of Example 1.

### Comparative Example 3

This comparative example differs from Example 1 in that the order of the two cross-linking reactions was reversed. Others were the same as those of Example 1.

### Performance tests

1. The collagen hydrogel fibers in Example 1 or Examples 1-4 were taken as examples to perform the following tests.
   (1) Macro-structure

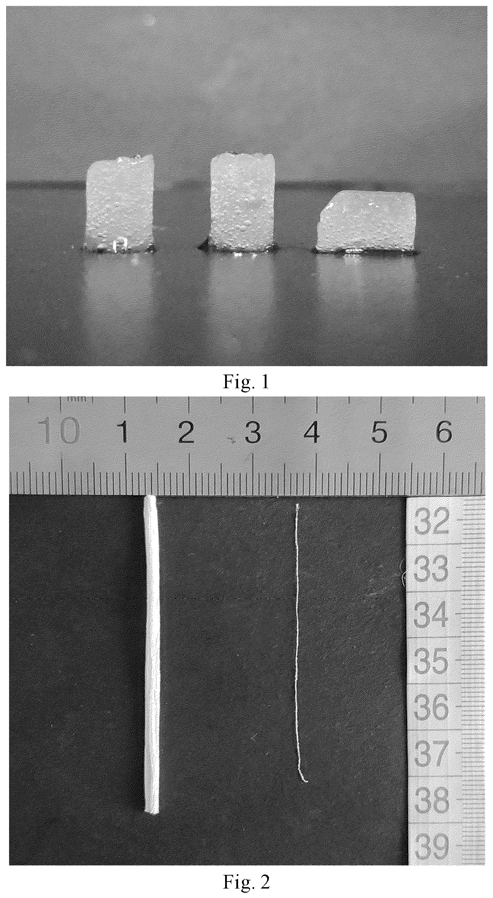

Fig. 1 shows the actual object of the collagen hydrogel in Example 1, and it can be seen from Fig. 1 that a large number of dense and uniform bubbles are distributed inside the bulk hydrogel, forming the pore structure.

The test sample used in Fig. 1 was prepared as follows: the product of the enzyme-catalyzed reaction after being centrifuged to remove bubbles in step (3) was immersed in NaHCO₃ solution instead of being squeezed with a syringe, and the preparation method is to facilitate the observation of the apparent morphology of the bulk hydrogel.

Fig. 2 shows the actual object of the collagen hydrogel fiber in Example 1, and it can be seen from Fig. 2 that the collagen hydrogel fiber has uniform linear structure, and the collagen ordered fiber scaffold prepared by collected collagen hydrogel fibers has a morphology similar to the real spinal cord.

### (2) Infrared test

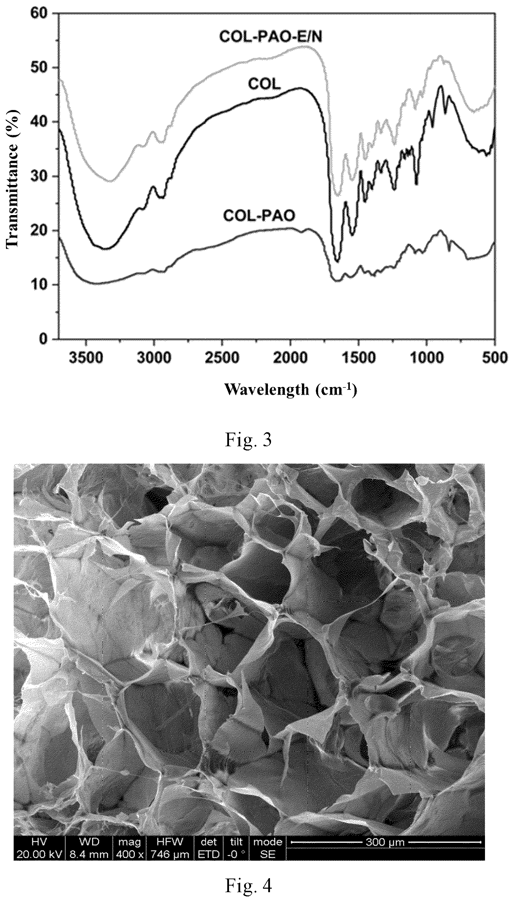

The pure collagen (COL) used in step (1), the product of the enzymatic cross-linking reaction (COL-PAO) in step (3) and the collagen hydrogel fiber (COL-PAO-E/N) obtained in step (5) of Example 1 were subjected to an infrared test, and the results are shown in Fig. 3. By comparing and analyzing the infrared spectra of collagen and enzymatic cross-linked collagen hydrogel, it can be seen that the C=O appearing near 1900 cm⁻¹ indicates that aldehyde groups were formed after the reaction, demonstrating the occurrence of Schiff base reaction; from the amide I and amide II bands near 1500-1650 cm⁻¹, it can be seen that the amide bands are strengthened for whether the enzymatic cross-linked collagen hydrogel or the collagen hydrogel after the secondary cross-linking; the absorption band near 2100 cm⁻¹ is assigned to -NH³⁺, and due to the decrease in free amino groups after the cross-linking reaction of collagen, the content of the protonated amino groups decreased, leading to a significant decrease of the peak intensity of the -NH³⁺ absorption band, indicating that the collagen underwent enzymatic cross-linking reaction and secondary cross-linking reaction.

### (3) Microscopic surface structure

The cross-section of the collagen hydrogel fiber in Example 1 was tested by a scanning electron microscope for surface morphology observation, and the result is shown in Fig. 4.

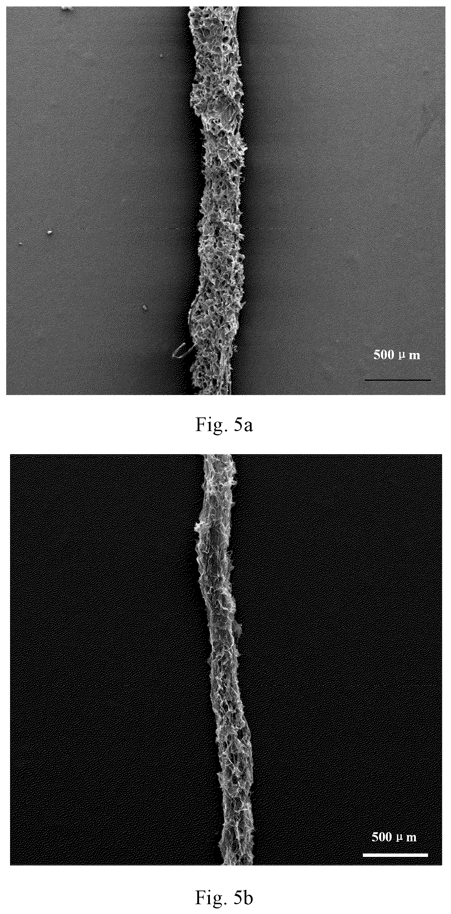

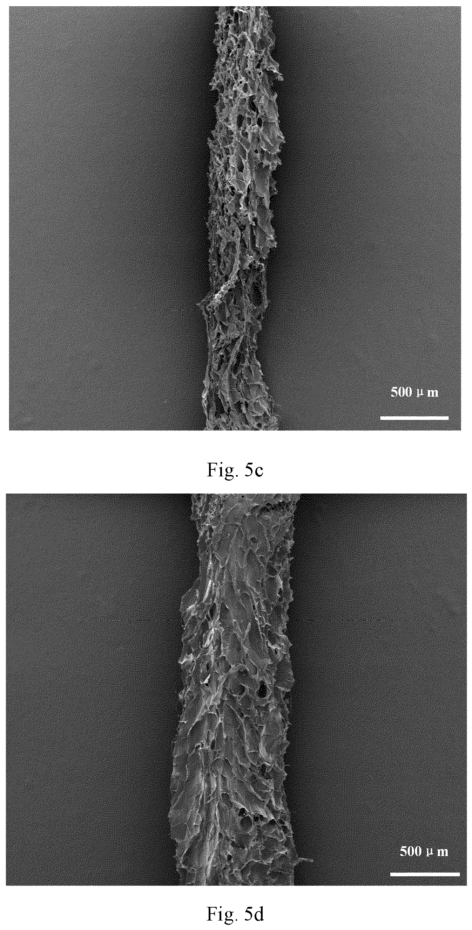

The collagen hydrogel fibers in Examples 1-4, i.e., the obtained collagen hydrogel fibers with different sizes, were subjected to SEM analysis for structure and morphology, and the results are shown in Fig. 5a, Fig. 5b, Fig. 5c and Fig. 5d.

SEM analysis shows that there are many cross-linking pores on the surface of the cured collagen hydrogel, indicating that the cross-linking reaction is carried out completely.

### (4) Time-sweep test

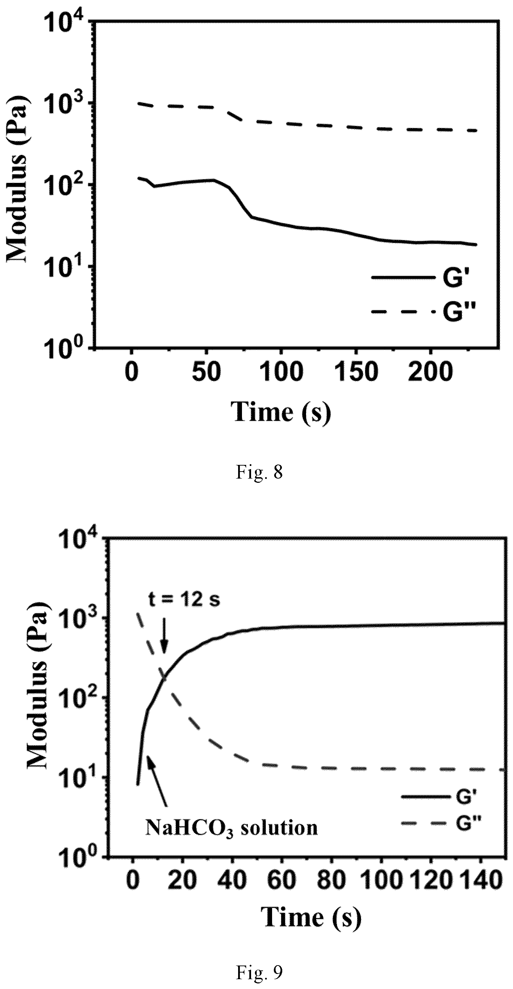

The primary cross-ling reaction of the collagen hydrogel fiber in Example 1 was subjected to a time-sweep test by a rotational rheometer, and the results are shown in Fig. 8. As shown by the results, at about 12 s, the elastic modulus (G') > the viscous modulus (G"); then G' is stable and greater than G", indicating that the collagen solution underwent gelation reaction at about 12 s and could be cured rapidly.

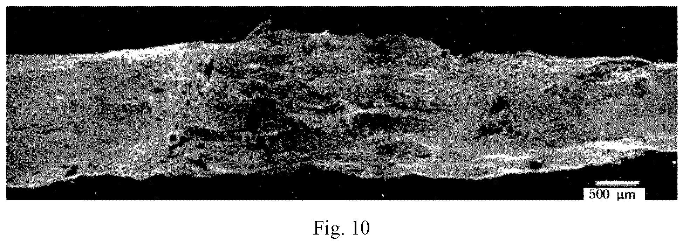

(5) Biological properties: the collagen hydrogel fibers were assembled into a fibrous scaffold (PCFS) with a diameter of about 2 mm and a length of about 4 cm to verify the injury repair function in a rat model of complete spinal cord injury, and immunofluorescence was labeled on the Tuj-1-positive neurons in the tissues of the injured area after 3 months of treatment. The results are shown in Fig. 10. It can be seen that PCFS can effectively promote endogenous neurogenesis, which is conducive to injury repair.

2. The collagen hydrogel fibers from Examples 1-12 and Comparative Examples 1-2 were subjected to the following tests.
(1) Mechanical properties: a 10 N sensor was used, a sample was placed on the tensile fixture of the sensor, and the test parameters were set according to the size of the sample; the stretching was stopped after the test curve changed abruptly, and the system automatically gave the mechanical property values;
   test parameters: a length of 10 mm, a diameter of 260 µm (Examples 1, 5, 6, 7, 8, 9, 10, and 11), a diameter of 210 µm (Example 2), a diameter of 420 µm (Example 3), a diameter of 860 µm (Example 4), and a compression rate of 0.5 mm/min.
(2) Fiber diameter: diameter of the fiber was observed and measured by scanning electron microscopy.

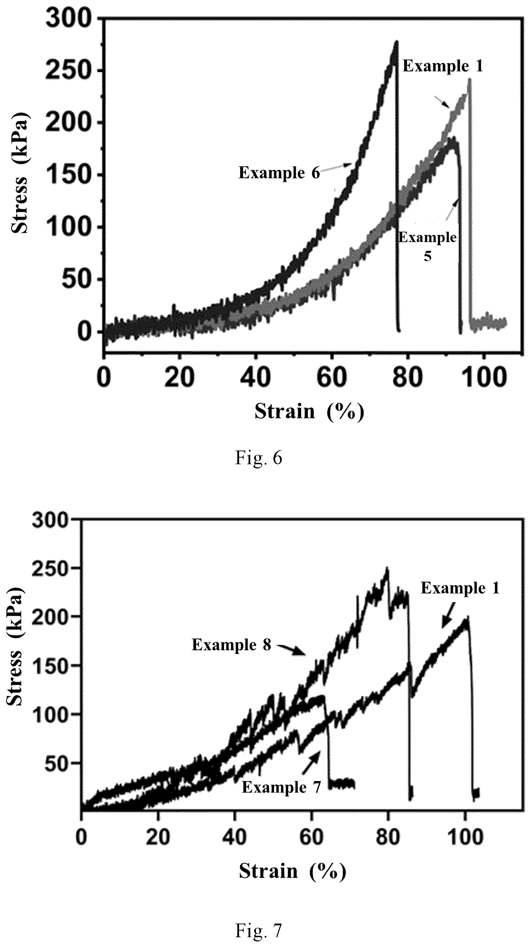

The test results are summarized in Table 1 and Figs. 6 and 7.

**Table 1**

| | Maximum elongation at break (%) | Maximum tensile stress (kPa) | Fiber diameter (µm) |
|---|---|---|---|
| Example 1 | 98.66 ± 2.56 | 260 ± 5.2 | 261.2 ± 8.74 |
| Example 2 | 89.63 ± 9.54 | 180.37 ± 9.16 | 199.59 ± 8.45 |
| Example 3 | 90.46 ± 10.15 | 268.01 ± 9.43 | 425.38 ± 14.27 |
| Example 4 | 87.08 ± 9.29 | 328.42 ± 10.64 | 860.91 ± 15.44 |
| Example 5 | 90.23 ± 1.23 | 176.02 ± 10.21 | 257.18 ± 9.95 |
| Example 6 | 79.13 ± 2.54 | 277.64 ± 16.02 | 259.15 ± 11.20 |
| Example 7 | 64.91 ± 1.2 | 118.60 ± 2.3 | 257.23 ± 8.63 |
| Example 8 | 86.70 ± 0.8 | 260.62 ± 17.63 | 264.87 ± 11.39 |
| Example 9 | 88.39 ± 1.83 | 210.83 ± 13.08 | 260.31 ± 7.12 |
| Example 10 | 95.61 ± 4.25 | 227.84 ± 10.91 | 261.92 ± 6.25 |
| Example 11 | 91.93 ± 11.22 | 280.53 ± 9.72 | 262.19 ± 6.33 |
| Example 12 | 45.53 ± 16.03 | 201.57 ± 12.33 | 275.18 ± 9.04 |
| Example 13 | - | - | - |
| Comparative Example 1 | 79.91 ± 10.26 | 189.56 ± 10.37 | 263.19 ± 5.37 |
| Comparative Example 2 | 50.71 ± 11.83 | 235.21 ± 7.39 | 269.91 ± 11.85 |
| Comparative Example 3 | 51.09 ± 7.82 | 241.17 ± 10.91 | 270.18 ± 16.18 |

Based on the analysis of Table 1, it can be seen that the collagen hydrogel fiber in the present application has a maximum elongation at break of 45.53 ± 16.03% - 98.66 ± 2.56%, a maximum tensile stress of 118.60 ± 2.3 kPa - 328.42 ± 10.64 kPa, and a fiber diameter within the range of 199.59 ± 8.45 - 860.91 ± 15.44 µm; the collagen hydrogel fiber in the present application has good mechanical strength and elastic properties, and an adjustable fiber diameter.

Based on the analysis of Examples 1, 5 and 6, it can be seen that, as shown by the result in Fig. 6, the collagen hydrogel fiber in the present application has good mechanical strength and elastic properties, and the mechanical strength of the collagen hydrogel gradually increases with the gradual increase of the initial substrate concentration within a certain range.

Based on the analysis of Example 1 and Examples 7 and 8, the performance of Examples 7 and 8 is not as good as that of Example1, and the result is shown in Fig. 7; it is proved that within a certain range, the mechanical properties of the obtained collagen hydrogel are changing with the changed substrate-enzyme concentration, and the best mechanical properties can be obtained after optimizing the enzyme-substrate concentration. In the present application, the concentration of the collagen solution at 30-50 mg/mL is more conducive to the preparation of high-performance collagen hydrogel.

Based on the analysis of Example 13 and Comparative Example 1, Example 13 failed in gelation without NaHCO₃, and the results are shown in Fig. 8 (Example 13) and Fig. 9 (Comparative Example 1), proving that NaHCO₃ provides the necessary conditions for enzymatic cross-linking.

Based on the analysis of Comparative Example 3 and Example 1, as shown by Table 1, it is proved that performing the PAO enzyme-catalyzed cross-linking first can increase the reversible change of the conformation of the molecular chain inside the collagen hydrogel, thus improving the fluidity of the collagen hydrogel, so that the elasticity of the hydrogel fiber increases, which expands the application range of the fiber.

The applicant declares that the present application illustrates the detailed solutions of the present application by the above examples, but the present application is not limited to the above detailed solutions, that is, the present application does not necessarily rely on the above detailed solutions to be implemented. Those skilled in the art should understand that any improvements of the present application, the equivalent substitution of each raw material for the product of the present application, the addition of auxiliary ingredients, and the selection of specific methods shall all fall within the protection scope and disclosure scope of the present application.

## Claims

1. A preparation method for a collagen hydrogel, which comprises the following steps:
(1) catalyzing oxidative deamination of collagen to generate unsaturated aldehyde functional groups by using amine oxidase for intramolecular or intermolecular cross-linking to complete a primary cross-linking; and
(2) subjecting the product from the primary cross-linking to a secondary cross-linking under the catalytic action of a carboxyl activator to obtain the collagen hydrogel.

2. The preparation method according to claim 1, wherein in step (1), the collagen and the amine oxidase are first prepared as a collagen solution and an amine oxidase solution, respectively, and then subjected to the catalytic reaction;
preferably, a concentration of the collagen solution is 20-60 mg/mL;
preferably, pH of the collagen solution is less than 7;
preferably, a solvent of the collagen solution comprises an organic acid solution;
preferably, a solvent of the collagen solution comprises an acetic acid solution;
preferably, a mass fraction of the acetic acid solution is 0.01%-30%.

3. The preparation method according to claim 2, wherein a concentration of the amine oxidase solution is 5-7 U/mL;
preferably, pH of the amine oxidase solution is 6.5-7.5;
preferably, the amine oxidase comprises any one or a combination of at least two of plasma amine oxidase, monoamine oxidase, diamine oxidase, or lysyl oxidase.

4. The preparation method according to any one of claims 1-3, wherein the secondary cross-linking is performed in a curing receiving solution containing the carboxyl activator;
preferably, pH of the curing receiving solution is 7-9;
preferably, the curing receiving solution comprises a weak alkaline solution;
preferably, the weak alkaline solution comprises any one or a combination of at least two of sodium bicarbonate, potassium bicarbonate, calcium bicarbonate, sodium carbonate or potassium carbonate.

5. The preparation method according to any one of claims 1-4, wherein the primary cross-linking is performed at a temperature of 4-37°C;
preferably, the primary cross-linking is performed for a period of 1 s-12 h.

6. The preparation method according to any one of claims 1-5, wherein the carboxyl activator comprises 1-(3-dimethylaminopropyl)-3-ethylcarbodiimide hydrochloride and/or N-hydroxysuccinimide;
preferably, a mass ratio of 1-(3-dimethylaminopropyl)-3-ethylcarbodiimide hydrochloride to N-hydroxysuccinimide is (1-10):1;
preferably, a concentration of the carboxyl activator in the curing receiving solution is 0.5-1 mg/mL.

7. The preparation method according to claim 6, wherein the secondary cross-linking is performed at a temperature of 4-37°C;
preferably, the secondary cross-linking is performed for a period of 0.1-12 h.

8. The preparation method according to any one of claims 1-7, which comprises the following steps:
(1) subjecting a collagen solution with a concentration of 20-60 mg/mL and pH of less than 7 and an amine oxidase solution with a concentration of 5-7 U/mL and pH of 6.5-7.5 to oxidative deamination by enzyme catalyzing at 4-37°C for 1 s-12 h to complete a primary cross-linking; and
(2) subjecting the product from the primary cross-linking to gelatinization and a secondary cross-linking at 4-37°C for 1 s-12 h in a curing receiving solution containing a carboxyl activator with pH of 7-9 to obtain the collagen hydrogel.

9. A collagen hydrogel, which is prepared by the preparation method according to any one of claims 1-8.

10. Use of the collagen hydrogel according to claim 9 in biomedical materials or scaffolds for tissue engineering.
